# EUROPÄISCHE PATENTSCHRIFT

(11) **EP 3 232 775 B1**
(45) Veröffentlichungstag und Bekanntmachung des Hinweises auf die Patenterteilung: **20.02.2019**
(21) Anmeldenummer: 15807654.7
(22) Anmeldetag: 08.12.2015
(51) Int. Cl.: A01M 7/00, A01M 9/00, A01C 21/00

(54) **VERFAHREN ZUR ERMITTLUNG EINES UNKRAUTANTEILS UND LANDTECHNIK-STEUEREINRICHTUNG**
METHOD FOR DETERMINING THE PORTION OF WEEDS AND AGRICULTURAL CONTROL DEVICE
PROCÉDÉ POUR LA DETERMINATION DE LA QUANTITÉ DES PLANTES ADVENTICES ET DISPOSITIF AGRICOL DE CONTROL

(30) Priorität: 17.12.2014 DE 102014226189
(43) Veröffentlichungstag der Anmeldung: 25.10.2017
(73) Patentinhaber: Continental Automotive GmbH, 30165 Hannover (DE)
(72) Erfinder: HOLLSTEIN, Armin, 93102 Pfatter (DE)
(86) Internationale Anmeldenummer: PCT/EP2015/078918
(87) Internationale Veröffentlichungsnummer: WO 2016/096526

(56) Entgegenhaltungen:
- WO-A1-99/19824
- DE-T2- 68 912 126
- US-A- 5 606 821
- US-A1- 2002 024 665

## Beschreibung

Zur Bekämpfung von unerwünschten Pflanzen ("Unkraut") zwischen Kulturpflanzen werden Herbizide eingesetzt, die über eine Feldfläche versprüht werden. Es ist bekannt, dass zur Einsatzoptimierung die Feldfläche gemäß dem Unkrautanteil (etwa der befallene Flächenanteil bezogen auf die gesamte betrachtete Fläche) mit einer variierenden Dosierung des Herbizids behandelt werden kann.

In der Druckschrift DE 689 12 126 T2 wird beschrieben, das Reflexionsvermögen eines Targetgebiets für bestimmte Bänder des elektromagentischen Spektrums zu erfassen, wobei die betreffenden erfassten Werte mit Werten einer bestimmten Nachschlagetabelle verglichen werden.

Zum einen ist die Erfassung eines Reflexionsvermögens fehleranfällig bei starker wechselnder Außenlichteinstrahlung. Zum anderen erfordert die Speicherung von und der Abgleich mit Nachschlagetabellen einen hohen Rechenaufwand, der mit der Güte der Adaption an Lichtverhältnisse ansteigt. Zudem müssen die Werte besagter Nachschlagetabellen auch den jeweiligen Wachstumszustand der Pflanzen berücksichtigen, was deren Umfang zusätzlich in die Höhe treibt.

Es ist eine Aufgabe der Erfindung, eine Möglichkeit aufzuzeigen, mit der auf einfache Weise die sensorgestützte Ausbringung von Herbiziden optimiert werden kann.

### Darstellung der Erfindung

Diese Aufgabe wird gelöst durch die Gegenstände der unabhängigen Ansprüche. Weitere Ausgestaltungen, Merkmale und Eigenschaften ergeben sich aus den Unteransprüchen sowie aus der folgenden Beschreibung.

Es ist vorgesehen, das Spektrum eines Betrachtungsabschnitts (der ggf. mit Pflanzenschutzmitteln behandelt wird) eines Feldes mit einem Spektrum eines Abschnitts des gleichen Feldes zu vergleichen. Der Referenzsensor blickt daher direkt auf einen Feldabschnitt, auf dem sich nur Nutzpflanzen (oder Nutzpflanzen mit einem zulässigen Unkrautanteil) befinden. Insbesondere blickt der Referenzsensor auf das gleiche Feld wie ein sogenannter Betrachtungsabschnitt-Sensor, der auf einen Betrachtungsabschnitt gerichtet ist, dessen Unkrautanteil ermittelt wird. Der Referenzsensor erfasst somit unter den gleichen Bedingungen (insbesondere Lichtbedingungen und Wachstumsstadium) die Spektralinformation wie derjenige Sensor, der auf den Abschnitt des Feldes (d.h. auf den Betrachtungsabschnitt) gerichtet ist, dessen Unkrautanteil zu bestimmen ist. Daher ergibt sich aus einem Vergleich der Spektralinformation der beiden Sensoren direkt der Unkrautanteil, insbesondere da andere Einflüsse (wie bei vorgefertigten Nachschlagetabellen) ausscheiden.

Der Referenzsensor ist insbesondere statisch. Der Referenzsensor kann und etwa am Rand des Feldes aufgestellt sein, etwa mittels einer Halterung wie ein Stativ. Es ergibt sich eine Ist-Spektralinformation eines Betrachtungsabschnitts und eine Referenz-Spektralinformation eines Referenzabschnitts, wobei die Ist- und die Referenz-Spektralinformation unter den gleichen Erfassungsbedingungen (etwa Beleuchtung, Entwicklungsgrad der Pflanzen, Lichtreflexion des Bodens etc.) ermittelt werden. In gleicher Weise ergeben sich für den Betrachtungsabschnitt und den Referenzabschnitt die gleichen Erfassungsbedingungen (oder zumindest Erfassungsbedingungen, die in einem bekannten Verhältnis zueinander stehen). Daher ist die Spektralerfassung präziser und ist gegenüber externer Beeinflussung (etwa die Änderung der Bildgebung bzw. der Beleuchtung) immun.

Es wird daher ein Verfahren zur Ermittlung eines Unkrautanteils in einem Betrachtungsabschnitt eines Feldes beschrieben, wobei eine Ist-Spektralinformation und eine Referenz-Spektralinformation erfasst wird. Die Ist-Spektralinformation wird mit mindestens einem Sensor erfasst, der auf den Betrachtungsabschnitt gerichtet ist, während die Referenz-Spektralinformation mittels eines vorzugsweise gleichartigen Sensors erfasst wird, der auf einen Referenzabschnitt gerichtet ist. Die Sensoren sind optische Sensoren und sind insbesondere in der Lage, die Intensität zumindest zweier Lichtwellenlängen für das Sichtfeld des Sensors zu erfassen. Das Sichtfeld des jeweiligen Sensors kann dem betreffenden Abschnitt entsprechen oder kann diesen (neben anderen Bildabschnitten) umfassen.

Der Referenzabschnitt und der Betrachtungsabschnitt sind Abschnitte des gleichen Feldes bzw. der gleichen Feldfläche, d.h. die Nutzpflanzen in beiden Abschnitten wurden zu selben Zeit ausgesät und befinden sich daher in der gleichen Wachstumsphase. Durch die örtliche Nähe ergeben sich die gleichen Erfassungsbedingungen, so dass unterschiedliche Lichtverhältnisse bei der Ermittlung des Unterschieds zwischen den Spektralinformationen herausfallen. Vorzugsweise werden die Ist-Spektralinformation und die Referenz-Spektralinformation in einem zeitlichen Abstand zueinander aufgenommen, der nicht größer als eine vorbestimmte Dauer ist. Diese Dauer ist derart bemessen, dass davon auszugehen ist, dass sich innerhalb des zeitlichen Abstands keine Änderung der Lichtverhältnisse ergeben, die zu unterschiedlichen Meßergebnissen (d.h. unterschiedlichen Unkrautanteilen) führen können. Die Dauer beträgt beispielsweise 1 oder 0.5 Sekunden. Sowohl die Ist-Spektralinformation als auch die Referenz-Spektralinformation kann vor der Ermittlung des Unterschieds gemittelt werden (etwa über ein Zeitfenster) oder einer Tiefpassfilterung unterzogen werden; zudem können Ausreißer der betreffenden Spektralinformation, die anhand von Veränderungsraten erkannt werden, die über einem vorgegebenen Schwellwert liegen, von der Erfassung oder von der weiteren Verarbeitung ausgenommen werden. Es können ferner Ausreißer bei den ermittelten Unterschieden von der weiteren Verarbeitung ausgenommen werden, wobei Ausreißer daran erkannt werden, dass der Betrag eines Unterschiedswerts eine vorgestimmte Grenze überschreitet. Etwa wenn (durch Einzelwolken oder Schattenwurf) der Referenzsensor abgeschattet ist und der Betrachtungsabschnittsensor nicht abgeschattet ist (oder umgekehrt), dann kann es zu Ausreißern kommen, die beim Ermitteln des Unterschieds bzw. beim Abbilden unterdrückt werden. Das Zeitfenster kann von der Geschwindigkeit abhängen, mit dem der Betrachtungsabschnitt über das Feld geführt wird. Je größer die Geschwindigkeit, desto kürzer ist vorzugsweise das Zeitfenster. Insbesondere ist die Meßrate, mit der Betrachtungsabschnitt-Sensor die Ist-Spektralinformation erfasst, von dieser Geschwindigkeit abhängig. Je größer die Geschwindigkeit ist, desto größer wird die Meßrate gewählt. Alternativ oder in Kombination hiermit kann die Meßrate bzw. das Zeitfenster von der Größe des Sichtfeldes des Betrachtungsabschnitt-Sensors abhängen. Ferner kann das Zeitfenster von der Fläche abhängen, die von einem Spritzmittelstrahl, der gemäß dem erfassten Unkrautanteil gesteuert wird, überdeckt ist. Je größer das Sichtfeld bzw. die vorangehend genannte Fläche, desto kürzer wird das Zeitfenster gewählt bzw. desto höher ist die Meßrate.

Der Sensor zur Erfassung des Betrachtungsabschnitts wird aufgrund dieser Funktion als Betrachtungsabschnitt-Sensor bezeichnet, und der Sensor zur Erfassung des Referenzabschnitts wird aufgrund dieser Funktion als Referenzabschnitts-Sensor bezeichnet. Es werden unterschiedliche Sensoren zur Erfassung des Referenzabschnitts und des Betrachtungsabschnitts verwendet.

Es wird ferner ein Unterschied zwischen der Ist-Spektralinformation und der Referenz-Spektralinformation ermittelt. Der Unterschied ist insbesondere ein mathematischer Differenzwert oder eine Differenzfunktion oder Differenzfolge, die für bestimmte Wellenlängen oder für bestimmte Wellenlängenintervalle die Differenz der Intensität (für eine Wellenlänge oder aufsummiert über das betreffende Wellenlängenintervall) wiedergibt. Der Unterschied kann auch durch eine Korrelation zwischen den Spektralinformationen gegeben sein, wobei eine hohe Korrelation einem geringen Unterschied entspricht und umgekehrt. Der Unterschied wird mittels einer vorgegebenen Abbildung auf den Unkrautanteil abgebildet. Die Abbildung kann als Zuordnung von Differenzwerten zu Unkrautanteilen in Sinne einer zweispaltigen Tabelle oder als Funktion, als Parametersatz einer Funktion, oder als Algorithmus realisiert sein. Ein erster Unterschied, der größer als ein zweiter Unterschied ist, ist mit einem Unkrautanteil verknüpft, der größer als der mit dem zweiten Unterschied verknüpfte Unkrautanteil ist. Mit anderen Worten entspricht die Abbildung einer steigenden Funktion, insbesondere einer monoton oder insbesondere streng monoton steigenden Funktion. Die Funktion kann kontinuierlich sein, oder kann (hinsichtlich des Unkrautanteils und des Unterschieds) wertdiskret sein. Es können insbesondere bei der Abbildung Interpolationsverfahren verwendet werden, falls nur wertdiskrete Unterschiede wertdiskreten Unkrautanteilen zugeordnet sind, der ermittelte Unterschied jedoch zwischen zwei vorliegenden (und mit Unkrautanteilen verknüpften) Unterschieden liegt. Diese Abbildung wird insbesondere von einem Mikroprozessor und einer Tabelle von Unterschieden und zugeordneten Unkrautanteilen realisiert, wobei ein erster Speicher(-abschnitt) für ein Programm eingerichtet zur Ausführung des Verfahrens und ein zweiter Speicher(-abschnitt) mit dem Mikroprozessor verbunden ist, und in dem zweiten Speicher die Unterschiede und die zugeordneten Unkrautanteile abrufbar abgelegt sind.

Vorzugsweise werden mehrere Betrachtungsabschnitte mittels mehrerer Betrachtungsabschnittsensoren erfasst. Jeder Betrachtungsabschnitt bzw. jede Ist-Spektralinformation jedes betreffenden Sensors wird von einer eigenen Vergleicher-Untereinheit und einer eigenen Abbildungs-Untereinheit verarbeitet. Es kann ferner (mindestens) eine ansteuerbare Düse für jeden Betrachtungsabschnitt (und somit auch für jeden Sensor bzw. für jede zugehörige Vergleicher- und Abbildungs-Untereinheit) vorgesehen sein. Für jede Düse kann ein (körperlich eigenständiges) Modul, vorzugsweise an der Düse, vorgesehen sein, wobei jedes Modul eine Vergleicher-Untereinheit und eine nachgeschalte Abbildungs-Untereinheit aufweist. Jedes Modul hat einen Ausgang (bzw. eine Untereinheit hiervon), mit dem eine zugehörige Düse angesteuert wird. In jedem Modul ist eine Vergleicher-Untereinheit, eine nachgeschalte Abbildungs-Untereinheit, der betreffende Ausgang und ein Eingang für die Referenz-Spektralinformation vorgesehen. Der an die Vergleicher-Untereinheit angeschlossene Beobachtungsabschnitt-Sensor ist vorzugsweise ebenso in dieses Modul integriert. Zudem kann die betreffende steuerbare Düse in dem Modul integriert sein. Wird etwa ein Spritzbalken als landwirtschaftliches Gerät verwendet, dann sind diese Module oder zumindest die Beobachtungssensoren entlang dieses Spritzbalkens aufgereiht insbesondere in einer oder in mehreren Reihen. Die Module befinden sich an der von dem jeweiligen Modul angesteuerten Düse, oder die betreffende Düse ist in dem betreffenden Modul integriert.

Hierin wird als Abbildungseinheit und Vergleichereinheit die (gesamte) Hardware bezeichnet, mit der die hier beschriebenen Vergleiche und Abbildungen ausgeführt werden. Diese Einheiten sind vorzugsweise jeweils körperlich verteilt, und insbesondere als körperlich verteilte Untereinheiten wie vorangehend beschrieben realisiert. Der Begriff "Einheit" ist daher nicht physisch sondern funktionsbezogen zu betrachten. Es kann ein Bus oder ein anderes Datennetz vorgesehen sein, mit dem die Referenz-Spektralinformation von einer betreffenden Eingangsschnittstelle (oder direkt von dem Referenzsensor) an die einzelnen Module bzw. Untereinheiten bzw. deren Eingänge verteilt wird.

Ein weiterer Aspekt betrifft die Auswertung der Spektralinformation, die vorzugsweise mehrere Spektralabschnitte betrifft. Es können mehrere Schwellwerte (insbesondere als Vektor) vorgesehen sein, die jeweils einen anderen Abschnitt des Spektrums betreffen. Der Unterschied ergibt sich aus einer Kombination der Vergleichsergebnisse (die sich mit den einzelnen Schwellwerten ergeben), etwa eine Summe der Vergleichsergebnisse oder eine Summe der Beträge der Vergleichsergebnisse. Es sind auch logische Kombinationen denkbar, wobei etwa die Anzahl der Schwellwertüberschreitungen ausgewertet wird und für den Unkrautanteil ausschlaggebend ist oder wobei die Überschreitungen mittels einer logischen Verknüpfung kombiniert werden. Bevorzugt werden die Vergleichsergebnisse gewichtet miteinander kombiniert, da einige Spektralabschnitte für den Unkrautanteil aussagekräftiger als andere sind.

Eine beispielhafte, einfache Realisierung der Abbildung ist es, einen Schwellwert (oder einen Schwellwertvektor) vorzugeben, mit dem der Unterschied (bzw. einem Unterschiedsvektor verglichen wird. (Vektoren werden verwendet, wenn für mehrere Spektralabschnitte jeweils ein Skalar als Unterschied ermittelt wird). Liegt der Unterschied (bzw. dessen Betrag im Falle eines vektoriellen Vergleichs) nicht über dem Schwellwert bzw. mit mehr als eine vordefinierte Marge über dem Schwellwert, wird ein Unkrautanteil ausgegeben, der kleiner ist als ein Unkrautanteil, der bei Überschreiten des Schwellwerts ausgegeben wird. In einem einfachen Fall wird ein Unkrautanteil von null ausgegeben, wenn der Schwellwert nicht erreicht wird, und es wird ein Unkrautanteil größer null (etwa eine Konstante, insbesondere eine eins) ausgegeben, wenn der Schwellwert erreicht oder überschritten wird. Wenn daher eine null als Unkrautanteil ausgegeben wird, so entspricht dies einem Beobachtungsabschnitt ohne Unkraut bzw. mit einem (vorgegebenen) akzeptablen Anteil an Unkraut, der keiner Behandlung bedarf. Wenn eine eins bzw. die Konstante abgegeben wird, so entspricht dies einem Beobachtungsabschnitt mit Unkraut in einem Maße, das eine Behandlung erfordert. Die eins (bzw. die Konstante) kann somit als Aktivierungssignal (für die Pflanzenbehandlung, d.h. für das Abgeben von Pflanzenschutzmitteln) angesehen werden, während bei einer Null die Pflanzenbehandlung bzw. allgemein die Abgabe von (Dünge- oder) Pflanzenschutzmittel nicht aktiv ist. Da der Unkrautanteil eng mit der Ansteuerung einer Feldspritze verknüpft ist (ein hoher Unkrautanteil ist gleichbedeutend mit der Notwendigkeit, die Feldspritze zu aktivieren), steht der Begriff "Unkrautanteil" neben dem tatsächlichen Mengen- oder Flächenanteil von Unkraut zwischen Nutzpflanzen auch für die Größen "Durchflussmenge", "Masse", "Durchflussgeschwindigkeit" oder insbesondere für "Flächenkonzentration" an Pflanzenschutzmittel.

Werden mehrere Betrachtungsabschnitt-Sensoren verwendet, insbesondere je Düse bzw. Spritzventil ein Sensor, kann als Eingangsgröße für die Abbildung das Verhältnis zweier Unterschiede zwischen Referenz- und Ist-Spektralinformationen verwendet werden.

Wie vorangehend bemerkt sind der Referenzabschnitt und der Betrachtungsabschnitt Bereiche desselben landwirtschaftlichen Feldes, die insbesondere nahe beieinander angeordnet sind, wobei sich die Abschnitte auch zumindest teilweise überlappen können. Stattdessen oder in Kombination hiermit können die Ist- und die Referenz-Spektralinformation wie vorangehend bemerkt in einem zeitlichen Abstand zueinander aufgenommen werden, der nicht größer als eine vorbestimmte Dauer ist. Sowohl durch die örtliche als auch durch die zeitliche Nähe gelten für beide Abschnitte bzw. für beide Spektralinformationen vergleichbare oder identische Aufnahmebedingungen, so dass die Differenz nur die spektrale Differenz wiedergibt und nicht zusätzlich Differenzkomponenten, die sich durch unterschiedliche Aufnahmebedingungen (etwa Beleuchtung, Entwicklungsgrad der Nutzpflanzen bzw. des Unkrauts, Reflexionen des Bodens etc.) ergeben. Beide Spektralinformationen werden für den gleichen Bodenzustand (feucht order trocken) erfasst. Es können etwa bei Abschattungen nur eines Sensors (d.h. eines Beobachtungsabschnitt-Sensors, während der Referenzsensors nicht abgeschattet ist, oder umgekehrt) die gemessenen Spektralinformationen unterdrückt werden bzw. nicht der weiteren Verarbeitung zugeführt werden, um Fehler zu vermeiden. Die Abschattung kann etwa durch einen Vergleich von erfasster Spektralinformation mit einer vorgegebenen Spektralinformation ermittelt werden. Hierbei wird der Unterschied der spektralen Verteilung von Sonnenlicht bei direkter Sonneneinstrahlung zu der spektralen Verteilung von Sonnenlicht bei indirekter / abgeschatteter / gestreuter Sonneneinstrahlung verwendet. Der Unterschied ergibt sich etwa durch die wellenlängenabhängige Dämpfung (d.h. Dispersion) etwa durch Wolken.

Der Referenzsensor kann an einer Feldfläche befestigt sein (etwa durch ein an der Feldfläche aufgestelltes Stativ oder eine in den Boden eingesteckte Halterung). Der Referenzsensor ist auf diese Feldfläche ausgerichtet. Über diese Feldfläche kann der Betrachtungsabschnitt bzw. der mindestens eine optische Betrachtungsabschnitt-Sensor geführt werden. Dadurch, dass beide Sensoren über die gleiche Feldfläche geführt werden, ergeben sich gleiche oder vergleichbare optische Erfassungsbedingungen. Hierbei ist der Referenzsensor ortfest, während der Betrachtungsabschnitt-Sensor beweglich ist, beispielsweise indem dieser auf einem (selbst angetriebenen) landwirtschaftlichen Nutzfahrzeug oder einem (gezogenen) mobilen landwirtschaftlichen Gerät befestigt ist. Der Begriff "ortsfest" schließt nicht aus, dass der Befestigungsort gewechselt werden kann.

Zwischen einem Zeitpunkt des Erfassens der Ist-Sensorinformation und einem Zeitpunkt des Erfassens der Referenzsensorinformation liegt nicht mehr als eine vorbestimmte Zeitdauer. Dadurch, dass die Erfassung der Spektren zeitlich korreliert ist, kann davon ausgegangen werden, dass diese unter den gleichen (oder unter ausreichend ähnlichen) Erfassungsbedingungen erfasst wurden. Es können Änderungen der Erfassungsbedingungen erfasst werden, die zwischen dem ersten und dem zweiten Zeitpunkt auftreten. Diese Änderungen (etwa wolkenfreie Witterung, die sich zu bewölkter Witterung ändert) können mittels einer vordefinierten Abbildung der Änderung auf einen Korrekturterm abgebildet werden, der mit dem Unterschied kombiniert wird oder der beim Abbilden des Unterschieds mitberücksichtigt wird. Vorzugsweise wird jedoch anstatt einer Korrektur die betreffende Information verworfen. Dies kann insbesondere die Referenz-Spektralinformation betreffen, wobei anstatt einer erneuten Messung eine vorangehend erfasste und zwischengespeicherte Spektralinformation als (aktuell) erfasste Spektralinformation herangezogen wird.

Die Ist-Spektralinformation und die Referenz-Spektralinformation charakterisieren zumindest ein Spektrumsintervall oder unterschiedliche Lichtwellenlängen. Insbesondere geben die Spektralinformationen ein Spektrumsintervall wieder, welches einen sichtbaren Bereich und/oder einen Infrarotbereich und/oder Ultraviolettbereich umfasst. Beispielsweise charakterisieren die Spektralinformationen ein Intervall, in dem die Farbe Gelb liegt (etwa 580 - 600 nm), ein Intervall, in dem die Farbe Grün liegt (etwa 525 - 575 nm), ein Intervall, in dem die Farbe Rot liegt (etwa 650 - 790 nm) und/oder einen Infrarotbereich von ca. 800 - 1000 nm (oder auch 900 - 1200 nm oder auch 1000 - 1500 nm und/oder einen Ultraviolettbereich, etwa einen Bereich innerhalb von UV-A, UV-B und/oder UV-C. Die Spektralinformation gibt für mehrere Wellenlängen oder Wellenlängenintervalle die Intensität des Lichts wieder.

Die Ist-Spektralinformation und die Referenz-Spektralinformation sind vorzugsweise jeweils wiedergegeben als ein Wert oder als eine Folge oder Tupel von Werten, die unterschiedliche Wellenlängen betreffen.

Da die Spektralinformation durch numerische Angaben wiedergegeben werden kann, ist eine Datenverarbeitung etwa mittels eines Mikroprozessors auf einfache Weise zu realisieren.

Die Ist-Spektralinformation und die Referenz-Spektralinformation werden erfasst mittels mindestens einem Farbsensor und/oder mittels mindestens einer elektronischen Kamera. Der Kamera ist vorzugsweise eine Farberfassungseinheit nachgeschaltet. Diese ermittelt die Farbwerte über das gesamte Bild oder über einen Bildabschnitt der Kamera. Hierbei kann die Farberfassungseinheit zur statischen Erfassung der Häufigkeit von unterschiedlichen Farbintervallen ausgebildet sein. Insbesondere kann die Farberfassungseinheit eine Histogrammfunktion aufweisen, mittels der die Häufigkeit von Bildpunkten mit verschiedenen Farbwerten erfasst wird. Während der Farbsensor nicht abbildet sondern die Spektralinformation über das gesamte Sichtfeld des Sensors ermittelt, wird zunächst von der Kamera ein (Farb-) Bild erstellt. Die Farbwerte der Bildpunkte der Kamera (oder eines Bildbereichs der Kamera) werden in der Farberfassungseinheit zusammengefasst zu dem Spektrum, das von der Spektralinformation wiedergegeben wird. Die (bildbezogene) Kamera wird durch die Farberfassungseinheit daher zum Farbsensor, der keine Bildauflösung bietet. Die Farberfassungseinheit mittelt daher über die Bildpunkte der Kamera, um zu einem Gesamtspektrum zu gelangen, das als Spektralinformation abgegeben wird.

Insbesondere zur Erfassung der Ist-Spektralinformation können mehrere Sensoren oder Kameras verwendet werden, deren Sichtfelder nebeneinander angeordnet sind. Die mehreren Sensoren sind etwa entlang einer Sprühleiste einer Feldspritze angeordnet, insbesondere äquidistant, beispielsweise mit einem Abstand von 20 - 100 cm, vorzugsweise mit einem Abstand von 40 - 60 cm. Dadurch kann eine landwirtschaftliche Vorrichtung, die mit dieser Vielzahl von Kameras oder Sensoren ausgestattet ist, für mehrere Betrachtungsabschnitte bzw. für einen großen Gesamt-Betrachtungsabschnitt die Ist-Spektralinformation erfassen. Der Sensor bzw. die Kamera kann das gesamte Sichtfeld aufnehmen, wobei etwa mittels eines berührungsempfindlichen Bildschirms ein Bildabschnitt eingegeben werden kann, für den die Spektralinformation erfasst wird, während Sensor- oder Bildinformation außerhalb des Bildabschnitts nicht berücksichtigt wird. Der berührungsempfindliche Bildschirm wird zur Auswahl des Sichtfelds der Betrachtungsabschnitt-Sensoren verwendet.

Ein Neigungssensor kann an dem mindestens einen Betrachtungsabschnitt-Sensor angeordnet sein oder der Neigungsensor kann an einem landwirtschaftlichen Gerät angeordnet sein, an dem sich der mindestens eine Sensor befindet, wobei der Neigungsensor eingerichtet ist, die Neigung des Geräts bzw. die Ausrichtung des Betrachtungsabschnitt-Sensors zu erfassen. Bei der Ansteuerung des landwirtschaftlichen Geräts (etwa eine Feldspritze) kann die Ausrichtung des Betrachtungsabschnitt-Sensors und/oder der Feldspritze gegenüber dem Feld berücksichtigt werden. Da der Sensor bzw. die Feldspritze bei zunehmender Neigung gegenüber dem Feld gemäß einer Tangens-Funktion auch auf eine zunehmende bzw. abnehmende Sichtfeldfläche gerichtet ist, ist diese Sichtfeldgrößenänderung bei der Auswertung bzw. Ansteuerung in Verbindung mit dem Abstand des Sensors von dem Zentrum der Neigungsbewegung (Drehachse) zu berücksichtigen.

Der Unkrautanteil bildet die Basis für die Auslösung eines Spritzmittelapplikationsvorgangs. Berücksichtigt wird dabei eine vom Benutzer vorgegebene Auslösegrenze, die einem noch zu tolerierenden Unkrautbesatz entspricht, ab dem Spritzmittel gespritzt werden soll. Diese Auslösegrenze kann durch den Schwellwert der Abbildungseinrichtung vorgegeben werden, sofern die Abbildung auf einem Vergleich mit einem (oder mehreren) Schwellwert(en) basiert.

Zudem werden ein Spritzbalkenwinkel und die Fahrtgeschwindigkeit (wie im nächsten Abschnitt dargestellt) berücksichtigt. Der Spritzbalkenwinkel ist der Winkel des Spritzbalkens oder einer anderen Halterung, an der der Beobachtungsabschnitt-Sensor befestigt ist. Der Spritzbalkenwinkel entspricht der Neigung und insbesondere dem Abstand des Sensors gegenüber dem Feld. Die Fahrtgeschwindigkeit bestimmt einen Vorhalt, mit dem der Weg kompensiert wird, den der Beobachtungsabschnitt zwischen Erfassung der Spektralinformation und dem Auftreffen des ausgebrachten Mittels auf dem Feld zurückgelegt wird. Daher kann die Düse um eine Zeitdauer verzögert angesteuert werden, wobei durch die Zeitdauer der Weg im Wesentlichen kompensiert wird.

Ferner ist die Geschwindigkeit zu berücksichtigen, mit der das landwirtschaftliche Gerät bzw. der mindestens eine Sensor über das Feld geführt wird. Bei einer ersten Neigung, die größer als eine zweite Neigung ist, wird die Feldspritze mit einem größeren (gemittelten) Durchfluss angesteuert als bei der zweiten Neigung. Die Abhängigkeit kann im Wesentlichen an eine Arctan-Funktion (oder deren Quadrat) angenähert sein. Bei einer ersten Geschwindigkeit, die größer als eine zweite Geschwindigkeit ist, wird die Feldspritze mit einem größeren (gemittelten) Durchfluss angesteuert als bei der zweiten Geschwindigkeit. Die Abhängigkeit kann im Wesentlichen linear sein.

Es können ferner Abschattungen (bzw. allgemein Aufnahmebedingungen) des Betrachtungsabschnitts und/oder des Referenzabschnitts ermittelt werden, etwa mittels des Sensors oder der Kamera (zur Erfassung des Betrachtungsabschnitts) selbst, oder mittels eines weiteren Lichtsensors. Bei unterschiedlichen Abschattungen (zwischen Betrachtungsabschnitt und Referenzabschnitt) kann die Ist- oder die Referenz-Spektralinformation oder auch der Unterschied zwischen der Ist- und der Referenz-Spektralinformationen durch arithmetische Kombination mit einer vorgegebenen Beleuchtungskorrekturvariablen angepasst werden. Die Beleuchtungskorrekturvariable ergibt sich aus der erfassten Abschattung (bzw. aus der Änderung der Aufnahmebedingungen), etwa über eine vorgegebene Abbildung. Diese Abbildung kann auf empirischen Daten beruhen und eine Beeinflussungskomponente wiedergeben, die sich durch unterschiedliche Aufnahmebedingungen ergibt. Vorzugsweise wird jedoch bei erfassten unterschiedlichen Abschattungen (d.h. Abschattung nur des Referenzsensors oder nur des mindestens einen Betrachtungsabschnitt-Sensors) der abgegebene Unkrautanteil nicht verändert. Mit anderen Worten werden bei teilweisen Abschattungen keine aktuellen Vergleiche aktueller Spektralinformationen durchgeführt. Stattdessen werden Unkrautanteile (bzw. diesen entsprechenden Ansteuersignale) verwendet, die vor der Erfassung der Abschattung verwendet wurden. Sind beide Sensoren abgeschattet oder wird die Abschattung aufgehoben, wird das Verfahren wie eingangs erwähnt durchgeführt.

Die Aufnahmebedingungen (d.h. Abschattung) können neben einer Erfassung mittels Sensor in einer spezifischen Ausführungsform über eine Eingabeschnittstelle eingegeben werden, um dem Benutzer die Gelegenheit zu geben, Informationen über unterschiedliche Aufnahmebedingungen (der Referenz-Spektralinformation gegenüber der Ist-Spektralinformation) einzugeben. Diese eingegebenen Aufnahmebedingungen werden bei der Auswertung der mittels Sensor erfassten Spektralinformationen berücksichtigt. Die Eingabeschnittstelle für die Aufnahmebedingungen kann etwa für den Betrachtungsabschnitt und für den Referenzabschnitt auswählbare Symbole oder Begriffe darstellen, die unterschiedliche Aufnahmebedingungen darstellen. Dadurch kann für jede Spektralinformation die betreffende Aufnahmebedingung eingegeben werden, so dass die genannten Änderungen sich durch Eingabe unterschiedlicher Aufnahmebedingungen unmittelbar ergeben. Die auswählbaren oder erfassbaren Aufnahmebedingungen können etwa sein: keine Bewölkung / leicht bewölkt / stark bewölkt / Tageslicht / Dämmerung / Niederschlag / Nebel / Boden feucht / Boden trocken / abgeschattet / direkte Sonneneinstrahlung. Da diese Aufnahmebedingungen Auswirkungen auf das Spektrum des eingestrahlten Lichts haben, kann durch diese Vorgehensweise ein resultierender Fehler zumindest teilweise kompensiert werden. Dies betrifft insbesondere die Eingabe ob nur der Referenzsensor oder nur der mindestens eine Eingabesensor abgeschattet ist oder auf einen sonnenbestrahlten Abschnitt (insbesondere wolkenfrei) gerichtet ist.

Weiterhin kann der statische Referenzsensor auf einen Referenzabschnitt ausgerichtet werden, welcher keinen Unkrautanteil aufweist, oder einen Unkrautanteil aufweist, der keine Behandlung erfordert, etwa indem der Referenzsensor manuell beweglich gelagert ist oder mittels einer versetzbaren Halterung (Stativ oder ähnliches) angeordnet ist.

Der Farbsensor zur Erfassung der Referenz-Spektralinformation (d.h. der Referenzsensor) kann mit einer Kamera gekoppelt werden, so dass ein Bild des Sichtfeldes des Farbsensors von der Kamera erfasst wird. Dadurch wird dem Nutzer der Referenzabschnitt als Bild dargestellt (etwa auf dem berührungsempfindlichen Bildschirm) und es wird erfasst, wenn der Nutzer zu dem aktuellen Referenzabschnitt die Referenz-Spektralinformation aufnehmen möchte. Der Referenzsensor und die damit (mechanisch und funktionell) gekoppelte Kamera sind auf das gleiche Sichtfeld ausgerichtet. Mittels der bildlichen Darstellung der Information, die der als Kamera ausgestaltete Referenzsensor abgibt, kann sich der Bediener über den Unkrautanteil des Referenzabschnitts vergewissern und ggf. einen anderen Referenzabschnitt auswählen (durch Umsetzen oder erneutes Ausrichten des Referenzsensors). Allgemein kann der der Referenzabschnitt derart gestaltet werden, dass dieser eine Obergrenze des Unkrautanteils aufweist, etwa indem eine noch tolerierbare Menge an Unkraut auf einen ansonsten unkrautfreien Abschnitt ablegt. Die Obergrenze markiert den Unkrautanteil, der noch keine Behandlung erfordert. Anstatt der Ablage von Unkraut kann eine Farbtafel auf den Referenzabschnitt abgelegt werden, wobei die Farbtafel den noch tolerierbaren Anteil an Unkraut darstellt.

Im Weiteren wird eine Landtechnik-Steuereinrichtung beschrieben, die insbesondere zur Ausführung des Verfahrens ausgestaltet ist. Die Landtechnik-Steuereinrichtung ist vorzugsweise eine Steuereinrichtung für Agrar- oder Landschaftspflege, insbesondere für eine Feldspritze oder für einen Düngerausbringer. Für die Verwendung eines Düngerausbringer sollte der Referenzsensor auf einem Feldbereich stehen, der sich in einem z. B. guten/oder schlechten Ernährungszustand befindet, etwa in einem Ernährungszustand, der eine Untergrenze darstellt, ab der Dünger auszubringen ist.

Der Betrachtungssensor bzw. die nachgelagerte Auswertung wie sie im Folgenden beschrieben ist, kann insbesondere ausgestaltet sein, anhand eines Abgleichs mit vorgegebenen Spektraldaten zu unterscheiden können, ob die gemessenen Abweichungen von den Referenzwerten auf Ernährungszustand oder Unkrautbesatz zurückzuführen sind. Dadurch kann vermieden werden, dass Pflanzenschutzmittel auf zu düngende Nutzpflanzen abgegeben wird, bzw. wird vermieden, dass zu Nutzpflanzen mit geringem Ernährungszustand als Unkraut eingeschätzt werden. Ferner kann dadurch vermieden werden, dass Dünger auf Unkraut ausgebracht wird, wobei insbesondere vermieden wird, dass ein hoher Unkrautanteil fälschlicherweise als Nutzpflanzen mit geringem Ernährungszustand eingeschätzt werden.

Die Steuereinrichtung verfügt vorzugsweise über einen Ansteuerausgang, über den die Steuereinrichtung in der Lage ist, die Menge bzw. den Durchsatz der anschließbaren Feldspritze oder des Düngerausbringers zu steuern.

Die Landtechnik-Steuereinrichtung hat mindestens einen optischen Betrachtungsabschnitt-Sensor, etwa mindestens ein Betrachtungsabschnitt-Sensor wie er hierin beschrieben ist. Vorzugsweise sind eine Vielzahl von Betrachtungsabschnitt-Sensor vorgesehen, die entlang einer Zeile oder entlang mehrerer paralleler Zeilen angeordnet sind. Insbesondere wird für jede Düse des landwirtschaftlichen Geräts (Feldspritze oder Düngerausbringer) ein Betrachtungsabschnitt-Sensor vorgesehen. Das jeweilige Gebiet, in das jede Düse Dünger oder Pflanzenschutzmittel abgibt, entspricht einem Sichtfeld (d.h. Betrachtungsabschnitt) eines Betrachtungsabschnitt-Sensors, der dieser Düse zugeordnet ist.

Die Landtechnik-Steuereinrichtung hat ferner einen optischen Referenzsensor, etwa einen Referenzsensor wie er hier beschrieben ist. Der Referenzsensor ist stationär und ist über eine drahtlose Übertragungsschnittstelle mit den im Weiteren beschriebenen Auswertungskomponenten (Vergleicher, Abbildung) verbindbar. Die Landtechnik-Steuereinrichtung weist daher eine Referenzsensor-Empfangsschnittstelle auf, die mit dem Referenzsensor wie beschrieben verbindbar ist. Der stationäre Referenzsensor kann als ein Teil der Landtechnik-Steuereinrichtung angesehen werden, wobei die Landtechnik-Steuereinrichtung auch ohne stationären Referenzsensor zur Realisierung der hier beschriebenen Erfindung geeignet ist, sofern diese die Referenzsensor-Empfangsschnittstelle aufweist.

Die Sensoren sind optische Sensoren und eingerichtet, Spektralinformationen des Sichtfelds des betreffenden Sensors zu erfassen.

Die Referenzsensor-Empfangsschnittstelle ist an dem gleichen Fahrzeug wie der Betrachtungsabschnitt-Sensor befestigt. Der Referenzsensor selbst ist stationär und über eine drahtlose Verbindung, etwa eine Funkverbindung, mit der Referenzsensor-Empfangsschnittstelle verbindbar bzw. verbunden.

Die Landtechnik-Steuereinrichtung weist ferner einen Vergleicher auf, der den Sensoren nachgeschaltet ist. Der Vergleicher ist eingerichtet, Ist-Spektralinformation des Betrachtungsabschnitt-Sensors mit Referenz-Spektralinformation des optischen Referenzsensors zu vergleichen. Der Vergleicher gegebenenfalls eingerichtet, Ist-Spektralinformation des mindestens einen Betrachtungsabschnitt-Sensors mit Referenz-Spektralinformation zu vergleichen, die über die Referenzsensor-Empfangsschnittstelle empfangen wird.

Vorzugsweise werden mehrere Betrachtungsabschnitt-Sensoren verwendet, die jeweils mit einer Vergleicher-Untereinheit des Vergleichers verbunden sind. Die Referenz-Spektralinformation oder ein daraus abgeleitetes Signal wird über einen Datenbus an jede Vergleicher-Untereinheit übermittelt. Der Datenbus verbindet die Referenzsensor-Empfangsschnittstelle mit den Vergleicher-Untereinheiten. Der Datenbus verteilt auf diese Weise die Referenz-Spektralinformation an die Vergleicher-Untereinheiten des Vergleichers. Jede Vergleicher-Untereinheit ist kann als eigenes (körperlich individuelles) Modul ausgestaltet, das insbesondere an der zugehörigen Düse angeordnet ist. Ein derartiges Modul kann ggf. neben dem zugehörigen Sensor auch eine zugehörige Abbildungsuntereinheit aufweisen. Vorzugsweise ist jedem Modul eine Düse zugeordnet. Jede Vergleicher-Untereinheit ist mit einem Betrachtungsabschnitt-Sensor verbunden, dessen Betrachtungsabschnitt (d.h. dessen Sichtfeld) auf einen Abschnitt gerichtet ist, in den auch die Düse gerichtet ist. Die Düse umfasst ein elektrisch steuerbares Ventil. Dieses wird (indirekt) von der zugehörigen Vergleicher-Untereinheit des Vergleichers angesteuert. Die Ansteuerung erfolgt über eine Abbildungseinheit bzw. mehrere Abbildungs-Untereinheiten der Abbildungseinheit, wie im Folgenden dargestellt ist.

Die Landtechnik-Steuereinrichtung verfügt ferner über eine derartige Abbildungseinheit, die dem Vergleicher nachgeschaltet ist. Die Abbildungseinheit ist eingerichtet, das Vergleichsergebnis des Vergleichers auf einen Unkrautanteil abzubilden und an einem Ausgang der Landtechnik-Steuereinrichtung abzugeben. Dieser Ausgang ist insbesondere der Ansteuerausgang. Die Abbildungseinheit kann eine Abbildung etwa in Form einer Look-up-Tabelle oder einer Näherungsformel aufweisen, ist jedoch vorzugsweise als Schwellwert-Vergleicher ausgestaltet. Die Abbildung ordnet unterschiedlichen Vergleichsergebnissen individuellen Unkrautanteilen zu. Basis für die Abbildung kann eine empirische Erfassung des Verhältnisses von Unkrautanteilen zu Unterschieden zwischen Ist- und Referenzspektren sein. Der Unkrautanteil kann als Flächenanteil in Sinne von Unkrautbewuchs / Nutzpflanzenbewuchs dargestellt sein, kann als Masseanteil Unkrautbewuchs / Nutzpflanzenbewuchs dargestellt sein, oder kann als eine weiterführende Größe etwa im Sinne der auszubringenden Pflanzenschutzmenge dargestellt sein, die sich unmittelbar aus dem fläche- oder massebezogenen Unkrautanteil ergibt. Insbesondere kann der Unkrautanteil unmittelbar als Ansteuergröße für die betreffende Düse ausgestaltet sein und somit direkt einer Ansteuergröße entsprechen.

Die Abbildungseinheit weist insbesondere mehrere Abbildungs-Untereinheiten auf. Für jede Düse bzw. für jeden Betrachtungsabschnitt-Sensor (d.h. für jeden Betrachtungsabschnitt) ist eine Abbildungs-Untereinheit vorgesehen. Jeder Vergleicher-Untereinheit ist eine individuelle Abbildungs-Untereinheit nachgeschaltet. Jeder Abbildungs-Untereinheit ist ein Ansteueranschluss einer zugehörigen Düse zugeordnet. Jede Abbildungs-Untereinheit kann als ein (körperlich eigenständiges) Modul ausgebildet sein. Vorzugsweise ist jeder Düse eine Abbildungs-Untereinheit und eine (dieser vorgeschalteten) Vergleicher-Untereinheit vorgeschaltet. Jede Abbildungs-Untereinheit und die zugehörige Vergleicher-Untereinheit können als ein (körperlich eigenständiges) Auswertungsmodul ausgestaltet sein. Jedem Auswertungsmodul ist eine zugehörige Düse nachgeschaltet, etwa über einen Ansteuerausgang des Auswertungsmoduls. Jedes Auswertungsmodul umfasst (mindestens) zwei Dateneingänge bzw. einen kombinierten Dateneingang eingerichtet zum Empfang von Ist- und Referenz-Spektralinformation, insbesondere einen Bus-Anschluss zur Verbindung mit der Referenz-Empfangsschnittstelle und einen Anschluss für den zugehörigen Betrachtungsabschnitt-Sensor (der wie auch die zugehörige Düse auf den betreffenden Betrachtungsabschnitt ausgerichtet ist. Es kann für jede Düse oder für jedes Auswertungsmodul ein weiterer Aktivierungseingang vorgesehen sein, der mit einer Benutzereingabeschnittstelle oder einem entsprechenden Eingabekanal verbunden ist, mittels dem der Bediener einen Aktivierungsbefehl bzw. Deaktivierungsbefehl für eine oder ein Gruppe von Düsen eingeben kann. Dieser Befehl wird von der Düse bzw. dem Auswertungsmodul unabhängig von dem betreffenden Unkrautanteil ausgeführt; der Aktivierungseingang ist somit gegenüber dem Auswertungsmodul priorisiert. Der Aktivierungseingang kann ferner als ein Steuereingang ausgebildet sein, wie er im Folgenden beschrieben ist.

Die Abbildungseinheit oder die Landtechnik-Steuereinrichtung kann einen weiteren Steuereingang aufweisen, mit dem sich Bediener- oder Steuervorgaben (etwa eine Grundmenge oder einen Grunddurchsatz) eingeben lassen. Die Landtechnik-Steuereinrichtung und insbesondere die Abbildungseinheit sind eingerichtet, das Abbildungsergebnis (d.h. den Unkrautanteil, der von der Abbildungseinheit ermittelt wird) mit den Bedienvorgaben zu kombinieren, um ein derart kombiniertes Steuersignal am Ausgang abzugeben. Hierzu kann die Landtechnik-Steuereinrichtung oder die Abbildungseinheit eine vorzugsweise ausgangsseitige Kombiniereinrichtung aufweisen, etwa ein Addierer, der beispielsweise über gewichtete Eingänge verfügen kann. Der Steuereingang gibt etwa eine Grundmenge bzw. ein Tastverhältnis zur Definition des Öffnungs-und Schließzustands der betreffenden Düse bzw. der betreffenden Gruppe von Düsen an.

Wie erwähnt kann die Landtechnik-Steuereinrichtung eine Referenzsensor-Empfangsschnittstelle aufweisen. Diese ist eingerichtet, mit einem (externen oder zur Landtechnik-Steuereinrichtung zugehörigen) optischen Referenzsensor über eine drahtlose Datenverbindung verbunden zu sein. Der vorangehend genannte Referenzsensor ist vorzugsweise ausgebildet wird ein hier beschriebener Referenzsensor.

Der optische Referenzsensor ist als statischer Sensor ausgebildet. Wie erwähnt kann der statische Referenzsensor als Komponenten angesehen werden, die extern zu der Landtechnik-Steuereinrichtung ist, oder kann in einer andere Ausführungsform als (körperlich nicht verbundenes) Teil der Landtechnik-Steuereinrichtung angesehen werden.

Der Referenzsensor-Empfangsschnittstelle bzw. dem optischen Referenzsensor oder auch dem Betrachtungsabschnitt-Sensor kann eine Farberfassungseinheit (bzw. eine Untereinheit hiervon) nachgeschaltet sein. Jeder Vergleicher-Untereinheit kann eine Farberfassungs-Untereinheit vorgeschaltet sein. Diese Untereinheiten können zusammen in dem genannten Auswertungsmodul integriert sein.

Ferner kann eine Verifizierungseinheit vorgesehen sein, die mit mehreren Vergleicher- oder Abbildungsuntereinheiten verbunden ist. Diese vergleicht insbesondere die Ist-Spektralinformationen der einzelnen Beobachtungsabschnitt-Sensoren und erfasst, wenn diese Unterschiede aufweisen, die größer als ein vorbestimmter Schwellwert sind. Damit können partielle Abschattungen erfasst werden, d.h. Abschattungen von einzelnen oder mehreren Beobachtungsabschnitten, die nicht für alle Beobachtungsabschnitte zutreffen. Die Verifizierungseinheit ist mittels des Schwellwerts (bzw. mittels eines entsprechenden Schwellwertvektors für mehrere Spektralabschnitte) eingerichtet, für Abschattung typische Spektraländerungen zu erfassen und somit zu ermitteln, ob ein Beobachtungsabschnitt abgeschattet ist, während ein anderer nicht abgeschattet ist. Die Verifizierungseinheit ist ferner ansteuernd mit den Abbildungs-Untereinheiten bzw. mit den Düsen verbunden, um bei einer erfassten partiellen Abschattung zumindest die betreffenden Düsen abzuschalten oder den vorangehend verwendeten Aktivierungszustand der Düse auch weiterhin zu halten, bis die Verifizierungseinheit keine partielle Abschattung mehr feststellt. Ohne erfasste partielle Abschattung greift die Verifizierungseinheit nicht in die Ansteuerung der Düsen bzw. in die Auswertung des Vergleichers bzw. der Abbildung ein. Ferner kann die Verifizierungseinheit eingerichtet sein, einen Mittelwert (bzw. einen Mittelwertvektor) über alle Ist-Spektralinformationen zu bilden im Sinne eines Scharmittelwerts über die Beobachtungssensoren. Diese kann insbesondere im Falle einer detektierten Abschattung als Ist-Spektralinformation den einzelnen Vergleicher-Untereinheiten vorgegeben werden. Anstatt eines Mittelwerts kann auch ein Median verwendet werden.

Die Figur 1 dient zur näheren Erläuterung einer Ausführungsform des vorangehend beschriebenen Verfahrens.

Die Figur 2 zeigt eine Ausführungsform der hier beschriebenen Landtechnik-Steuereinrichtung mit einigen optionalen Merkmalen.

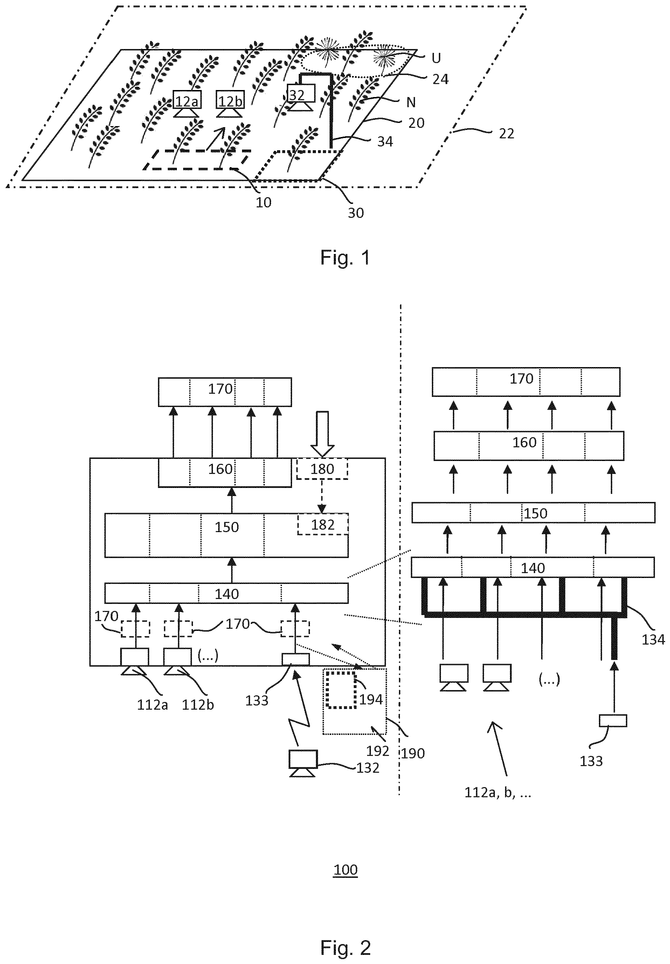

In der Figur 1 ist eine Anwendungsumgebung dargestellt und dient zur Erläuterung einer Ausführungsform des Verfahrens zur Ermittlung eines Unkrautanteils.

In einem Betrachtungsabschnitt 10, der über ein landwirtschaftlich betriebenes Feld 20 geführt wird, befinden sich Nutzpflanzen N und Unkraut U, wobei der Anteil des Unkrauts mit verschieben über das Feld 20 variiert. Das Feld 20 ist Teil einer Bodenfläche 22. In Bereichen, in denen sich Unkraut U befindet, etwa im Bereich 24, soll mehr Pflanzenschutzmittel ausgebracht werden als in Bereichen, in denen sich weniger oder kein Unkraut befindet.

Hierzu wird der Betrachtungsabschnitt 10 über das Feld 20 geführt, indem mindestens ein Betrachtungsabschnitt-Sensor, hier beispielhaft durch zwei Betrachtungsabschnitt-Sensoren 12a, b, über das Feld geführt werden, etwa mittels einer Zugmaschine oder eines Anhängers. Der Betrachtungsabschnitt 10 ergibt sich durch die Sichtfelder der Betrachtungsabschnitt-Sensoren 12a, b, die auf das Feld 20 gerichtet sind. Die Betrachtungsabschnitt-Sensoren 12a, b sind optische Sensoren und ermitteln Ist-Spektralinformation für Licht, das von dem Feld 12 a, b in die Sensoren zurückgeworfen wird. Zur Auswertung der Ist-Spektralinformation wird daher das Sonnenlicht oder anderes Umgebungslicht verwendet. Insbesondere sind keine Lichtquellen vorgesehen, die ausschließlich zur Auswertung der Spektralinformation verwendet werden. Die Spektralinformation bezieht sich auf sichtbares Licht und kann ferner UV- oder IR-Spektralanteile umfassen.

Das sich das zur Auswertung verwendete Licht abhängig von den Witterungsbedingungen ändert und damit auch das Spektrum des auf die Bodenfläche 22 veränderlich ist, wird Referenz-Spektralinformation mittels eines optischen Referenzsensors 32 erfasst, der auf einen Referenzabschnitt 30 der gleichen Bodenfläche 22 gerichtet ist. Der Unterschied zwischen der Ist-Spektralinformation und der Referenz-Spektralinformation wird ermittelt und aus dem Unterschied wird auf den Unkrautanteil geschlossen. Hierbei wird insbesondere eine Abbildung verwendet, die mehreren Unterschieden unterschiedliche Unkrautanteile zuordnet. Anstatt einer Zuordnung zu Unkrautanteilen kann direkt auf eine Steuergröße (Durchflussmenge, Masse, Durchflussgeschwindigkeit) einer Feldspritze, eines Düngerausbringers oder eines anderen landwirtschaftlichen Arbeitsgeräts abgebildet werden. Diese Steuergröße wird insbesondere mit einer Bediener- oder Steuervorgaben (etwa eine Spritzdauer, eine Grundmenge oder ein Grunddurchsatz) kombiniert, bzw. diese Bediener- oder Steuervorgaben wird gemäß der eingangs genannten Steuergröße, die sich aus dem Unterschied ergibt, verändert und derart verändert an einem Ausgang abgegeben, vgl. Figur 2. Dieser Ausgang ist zur ansteuernden Verbindung mit dem landwirtschaftlichen Arbeitsgerät ausgebildet und ist eingerichtet, entsprechende Ansteuersignale an das Arbeitsgerät abzugeben. Vorzugsweise gibt der Ausgang mehrere Signale ab, insbesondere ein Ansteuersignal für jede Düse des Arbeitsgeräts, vgl. Fig. 2.

Eine Veränderung des Lichteinfalls auf das Feld 20 (und somit des Spektrums des Lichts) führt zu einer Änderung, die gleichermaßen die Referenz-Spektralinformation wie die Ist-Spektralinformation betrifft. Da diese sich gleichermaßen mit der Veränderung des Lichteinfalls ändern, hebt sich bei der Bildung des Unterschieds diese Veränderung auf. Die Referenz-Spektralinformation und die Ist-Spektralinformation betreffen die gleiche Bodenfläche 22 und insbesondere das gleiche Feld 22 und sind somit dem gleichen Lichteinfall ausgesetzt.

Der Referenzabschnitt 30 ist Teil des Felds 20, über den der Betrachtungsabschnitt 10 geführt wird. Alternativ kann der außerhalb des Felds 20 sein, jedoch auf der gleichen Bodenfläche 22 vorgesehen sein wie das Feld 20.

Der Referenzsensor 32 ist teilweise oder vorzugsweise vollständig auf das Feld 20 gerichtet sein und ist statisch (d.h. ortsfest) . Der Referenzsensor 32 kann etwa mit einem Stativ oder einer in das Feld eindringenden Halterung 34 ortsfest gehalten werden.

Der Referenzsensor 32 ist zur drahtlosen Übermittlung der Referenz-Spektralinformation eingerichtet sein. Der Referenzsensor 32 weist eine drahtlose Datenübertragungsschnittstelle auf, über die die Referenz-Spektralinformation übertragen wird, insbesondere an eine Referenzsensor-Empfangsschnittstelle (vgl. Bezugszeichen 133 der Figur 2), um einen Vergleich der Referenz-Spektralinformation mit Ist-Spektralinformation an Bord der der Zugmaschine bzw. dem Arbeitsgerät (insbesondere in der Landtechnik-Steuereinrichtung, wie sie mit Bezugszeichen 100 in der Figur 2 dargestellt ist) zu ermöglichen.

Die Figur 2 zeigt links der senkrechten strichpunktierten Linie eine Übersicht über eine Landtechnik-Steuereinrichtung 100 und rechts der senkrechten strichpunktierten Linie Details zum Datenfluss bei Unterteilung einiger folgend dargestellten Komponenten in einzelne Untereinheiten.

Die Landtechnik-Steuereinrichtung 100 ist mit mindestens einem optischen Betrachtungsabschnitt-Sensor ausgebildet, etwa den hier dargestellten zwei Betrachtungsabschnitt-Sensoren 112a, b. Die Verwendung mehrerer Betrachtungsabschnitt-Sensoren 112a, b hat den Vorteil, dass ein breiterer Bereich abgedeckt werden kann. Das Sichtfeld jedes Sensors, d.h. jeder einzelne Beobachtungsabschnitt ist auf einen Abschnitt gerichtet, in den eine zugehörige Düse des landwirtschaftlichen Geräts 170, etwa eine Feldspritze, gerichtet ist.

Die mehreren Sensoren 112 a, b werden vorzugsweise einzeln bzw. mit individuellen Modulen (umfassend eine Vergleicher-Untereinheit und eine Abbildungs-Untereinheit) ausgewertet. Die Ist-Spektralinformationen der Betrachtungsabschnitt-Sensoren 112 a, b werden einzeln verwendet und jeweils mit der Referenz-Spektralinformation des Referenzsensors 132 verglichen.

Neben den Betrachtungsabschnitt-Sensoren 112 a, b ist mindestens ein optischer Referenzsensor 132 bzw. eine Referenzsensor-Empfangsschnittstelle 133 Teil der Landtechnik-Steuereinrichtung 100. Die Schnittstelle 133 ist an dem gleichen Fahrzeug (bzw. an der gleichen Zugmaschine bzw. an dem gleichen Arbeitsgerät) befestigt wie die Betrachtungsabschnitt-Sensoren 112 a, b.

Der optische Referenzsensor 132 ist ortsfest, entsprechend dem Referenzsensor 32 der Figur 1. Die Referenzsensor-Empfangsschnittstelle 133 ist mobil und eingerichtet, Daten, die die Referenz-Spektralinformation wiedergeben, von dem ortsfesten Referenzsensor 132 drahtlos zu empfangen. Der ortsfeste Referenzsensor 132 kann als Teil der Landtechnik-Steuereinrichtung 100 angesehen werden, wie es in Figur 2 dargestellt ist.

Alternativ ist nur die Referenzsensor-Empfangsschnittstelle und nicht der ortsfeste Referenzsensor physikalischer Bestandteil der Landtechnik-Steuereinrichtung, wobei die Referenzsensor-Empfangsschnittstelle die Aufgabe übernimmt, die Referenz-Spektralinformation zur Ausführung des Vergleichs zu beschaffen. Das Fahrzeug bzw. die Zugmaschine bzw. das Arbeitsgerät sind vorzugsweise nicht Teil der Landtechnik-Steuereinrichtung 100 sondern dienen nur zur Erläuterung des physischen Verhältnisses der Sensoren und untereinander bzw. der Sensoren / der Schnittstelle zu dem Feld.

Der Vergleicher 140 ist den Sensoren 112a, b und 132 bzw. der Schnittstelle 133 nachgeschaltet. Der Vergleicher 140 ist eingerichtet, Ist-Spektralinformation des mindestens einen Betrachtungsabschnitt-Sensors 112a, b mit Referenz-Spektralinformation des optischen Referenzsensors 132, 132' zu vergleichen. Der Vergleicher 140 ist in einzelne Untereinheiten aufgeteilt, wie mit den Punktlinien dargestellt ist. Jede Untereinheit ist an der betreffenden Düse des landwirtschaftlichen Geräts 170 befestigt oder ein einer Halterung, die die Düse trägt. Das landwirtschaftliche Gerät 170 ist in Unterabschnitte unterteilt, die einzelnen Düsen oder Düsengruppen entsprechen.

Der Vergleicher 140 ist insbesondere eingerichtet, Ist-Spektralinformation mit Daten zu vergleichen, die an der Schnittstelle 133 empfangen werden.

Eine Abbildungseinheit 150 der Landtechnik-Steuereinrichtung ist dem Vergleicher 140 nachgeschaltet. Die Abbildungseinheit 150 ist eingerichtet, das Vergleichsergebnis (oder bei sensorindividueller Verarbeitung der Ist-Spektralinformation die Vergleichsergebnisse) des Vergleichers an einem Ausgang 160 der Steuereinrichtung 100 abzugeben. Der Ausgang 160 ist eingerichtet, dass ein Ansteuereingang eines nachgeordneten Arbeitsgeräts (etwa eine Feldspritze) angeschlossen werden kann.

Die Abbildungseinheit 150 ist in einzelne Untereinheiten aufgeteilt, wie mit den Punktlinien dargestellt ist. Jede Untereinheit ist an der betreffenden Düse des landwirtschaftlichen Geräts 170 befestigt oder ein einer Halterung, die die Düse trägt. Vorzugsweise sind die Untereinheit der Abbildungseinheit 140 und die Untereinheit des Vergleichers 150 jeweils als ein Modul zusammen ausgeführt, das an der zugehörigen Düse oder an deren Halterung angebracht ist. Der Ausgang 160 gibt mehrere Signale ab, insbesondere ein Ansteuersignal für jede Düse des Arbeitsgeräts, wie es mittels der Unterteilung anhand der gestrichelten Linien dargestellt ist.

Die Landtechnik-Steuereinrichtung 100 kann einen weiteren Steuereingang 180 aufweisen, mit dem sich Bediener- oder Steuervorgaben (etwa eine Grundmenge, eine Grundintensität oder einen Grunddurchsatz) eingeben lassen. Eine Kombiniereinrichtung 182 empfängt die Bediener- oder Steuervorgaben (dargestellt durch den Doppelpfeil in der oberen Bildhälfte) und ist eingerichtet, das Abbildungsergebnis mit den an dem Steuereingang 180 empfangenen Daten arithmetisch oder logisch zu kombinieren. Dadurch ergibt sich am Ausgang 160 ein kombiniertes Steuersignal, das sich aus den Bediener- oder Steuervorgaben und dem Abbildungsergebnis (d.h. dem Unkrautanteil) zusammensetzt. Diese Kombination kann insbesondere als Modifikation der Bediener- oder Steuervorgaben betrachtet werden. Da der Unkrautanteil unmittelbar mit einer Änderung der Menge bzw. des Durchsatzes etwa einer Feldspritze verknüpft ist, lassen sich diese Größen im Sinne einer Modifikation oder Korrektur der Bediener- oder Steuervorgaben kombinieren.

Insbesondere ist die Kombiniereinrichtung 182 als Multiplikator ausgestaltet, so dass mit der Steuervorgabe die Spritzmenge eingestellt wird, mit dem im Falle eines hohen Unkrautanteils (bzw. geringem Entwicklungsgrad der Pflanze) gespritzt bzw. Dünger ausgebracht wird. Wird ein geringer Unkrautanteil oder kein Unkrautanteil ermittelt, so dass anhand des Abbildungsergebnisses für sich keine Abgabe vorgesehen wäre, würde auch eine Multiplikation mit einer Steuervorgabe dazu führen, dass keine Abgabe von Pflanzenschutzmittel (oder Dünger) angesteuert wird.

Falls die Kombiniereinrichtung 182 als ein Addierer ausgeführt ist, kann den Bediener- oder Steuervorgaben, die numerisch eine (Grund-)Menge oder einen (Grund-)Durchsatz von Pflanzenschutzmittel wiedergeben, eine numerische Darstellung des Unkrautanteils hinzuaddiert werden, so dass sich die auszubringende Menge an Pflanzenschutzmittel erhöht, wenn der Unkrautanteils erhöht ist, oder umgekehrt. Mit anderen Worten wird bei einem ersten Unkrautanteil die Bediener- oder Steuervorgabe mehr erhöht als bei dem zweiten Unkrautanteil, der kleiner als der erste Unkrautanteil ist. Ferner wird bei einem ersten Unterschied zwischen den Spektren die Bediener- oder Steuervorgabe mehr erhöht als bei dem zweiten Unterschied, der geringer ausfällt als der erste Unterschied. Ein hoher Unkrautanteil entspricht einer langen Spritzdauer bzw. intensiven Abgabe von Pflanzenschutzmittel (oder Dünger), während ein im Vergleich hierzu geringerer Unkrautanteil einer kürzeren Spritzdauer oder weniger intensiven Abgabe entspricht.

Dem mindestens einen optischen Betrachtungsabschnitt-Sensor 112a, b und/oder der Referenzsensor-Empfangsschnittstelle 133 bzw. dem optischen Referenzsensor 132' ist eine Farberfassungseinheit 170 nachgeschalte, wenn der betreffende Sensor als Kamera bzw. bildgebendes System ausgestaltet ist. Die Farberfassungseinheit 170 befindet sich zwischen dem Sensor 112 a, b; 132, 132' bzw. der Schnittstelle 133 und dem Vergleicher 140. Hierbei kann die Empfangsschnittstelle 133, der Referenzsensor 132, 132' und/oder der mindestens eine optische Betrachtungsabschnitt-Sensor 132 zur Aufnahme eines elektronischen Bildes bzw. zum Empfang von Bildinformation ausgebildet sein. Die Farberfassungseinheit 170 kann als Teil der Kamera ausgebildet sein, die auf dieses Weise als Farbsensor umfunktioniert wird.

Die Landtechnik-Steuereinrichtung 100 kann einen Bildschirm 190 aufweisen. Der Bildschirm 190 kann dem Referenz-Sensor 132 (bzw. der Schnittstelle 133) nachgeschaltet sein, wobei in diesem Fall als der Referenzsensor 132 als Kamera ausgebildet ist. Dies ermöglicht eine optische Kontrolle des Referenzabschnitts durch den Bediener, der ggf. über eine damit verknüpfte Eingabevorrichtung 182 einen Bildausschnitt 194 auswählen kann, etwa um den noch tolerierbaren Unkrautanteil zu definieren. Der Bildschirm weist hierzu allgemein eine Eingabevorrichtung auf, die in der Fig. 2 als berührungsempfindliche Schicht 192 dargestellt ist, die jedoch auch durch ein Eingabegerät wie eine Tastatur, eine Computermaus, ein Trackball oder ein Lichtgriffel realisiert sein kann. Die Referenz-Spektralinformation bezieht sich insbesondere nur auf den Abschnitt 194, falls ein solcher ausgewählt wird. Die berührungsempfindliche Schicht 192 und der darunterliegende Bildschirm 190 bilden zusammen eine berührungsempfindliche Anzeige.

Insbesondere der Sensor 132 ist als elektronische Kamera ausgestaltet, die in der Lage sind, Farbspektren des sichtbaren Lichts bis hin in den Infrarotbereich zu erfassen. Die Farberfassungseinheit 170 ist in der Lage, die einzelnen Pixel-Farbinformationen des betreffenden Bildes statistisch auszuwerten, um für das gesamte Bild oder auch nur für einen Bildausschnitt das Spektrum bzw. die Spektrumsinformation zu ermitteln. Hierbei kann etwa ein Histogramm der Farbwerte für alle betreffenden Pixel von der Farberfassungseinheit 170 erstellt werden, wobei das Histogramm (oder Teile hiervon) der Spektrumsinformation entspricht. Gegebenenfalls können auch die Sensoren 112a, b als Kameras ausgebildet sein, sofern diese eine ausreichende Erfassungsrate aufweisen.

Es sollte ferner ein Neigungssensor vorgesehen sein, der die Neigung der Sensoren 112 a, b und 132 bzw. des landwirtschaftlichen Geräts erfasst. Schwenkt das landwirtschaftliche Gerät (an dem die Sensoren 112 a, b befestigt sind) nach oben, so wird diese Bewegung und die sich ergebende Änderung der Ausrichtung der Sensoren berücksichtigt. Ebenso sollte die Landtechnik-Steuereinrichtung 100 einen Geschwindigkeitseingang aufweisen, der eingerichtet ist, ein Geschwindigkeitssignal zu empfangen, das die Geschwindigkeit wiedergibt, mit der die Betrachtungsfeldsensoren 112 a, b über das Feld geführt werden. An dem Geschwindigkeitseingang kann ein eigener, insbesondere Radar-basierter Geschwindigkeitssensor angeschlossen sein, der die Geschwindigkeit des Betrachtungsabschnitts über Grund ermittelt. Alternativ kann ein CAN-Bus des Fahrzeugs angeschlossen sein, in dem zahlreiche Daten, insbesondere Geschwindigkeitsinformation, zwischen Komponenten des Fahrzeugs übertragen werden. Im letztgenannten Fall wird somit der fahrzeugeigene Geschwindigkeitssensor genutzt.

Falls die Mitte des Gesichtsfelds der Betrachtungsfeldsensoren 112 a, b und die Ausrichtung der Spritzen bzw. des landwirtschaftlichen Geräts auseinanderfallen, wird eine Verzögerung bei der Umsetzung des ermittelten Unkrautanteils (d.h. bei der Ansteuerung der Düsen) eingeführt, die von dieser Geschwindigkeit abhängt. Dadurch wird der Weg berücksichtigt (bzw. kompensiert), der zurückgelegt wird zwischen Erfassung des Betrachtungsabschnitts und dem Zeitpunkt, an dem das landwirtschaftliche Gerät bzw. die Düse(n) über diesen Betrachtungsabschnitt geführt werden.

Die Bildhälfte rechts der strichpunktierten Linie der Fig. 2 zeigt in Detail die Betrachtungssensoren 112 a, b (beispielhaft), die gemäß den dargestellten Pfeilen die jeweilige Ist-Spektralinformation einzelnen Untereinheiten des Vergleichers 140 (siehe Punktlinienunterteilung) zuführen. Die Empfangsschnittstelle 133 empfängt die Referenz-Spektralinformation und verteilt diese über den Bus 134 (dargestellt durch dickere Linien) an die einzelnen Untereinheiten des Vergleichers 140. Die Untereinheiten des Vergleichers 140 (die auch als Vergleicher-Untereinheiten bezeichnet werden können) sind jeweils Untereinheiten der Abbildungseinheit 150 vorgeschaltet, wie es mit den Pfeilen dargestellt ist. In einem einfachen Fall umfasst die Abbildungseinheit 150 einen Eingang für einen Schwellwert oder einen Schwellwertvektor, an dem die Empfindlichkeit der Abbildungseinheit (d.h. die Sensitivität gegenüber Unkraut) eingestellt wird. Der Abbildungseinheit 150 ist ein Ausgang 160 nachgeschaltet, über den einzelne Ansteuersignale abgegeben werden, wie es durch die Unterteilung (siehe Punktlinien) dargestellt ist. Der Ausgang 160 kann in einzelne Untereinheiten unterteilt sein, die insbesondere körperlich eigenständig sind. Es können Untereinheiten des Vergleichers, der Abbildungseinheit und des Ausgangs, die dem selben Betrachtungsabschnitt-Sensor 112a zugeordnet sein, als ein (körperlich eigenständiges) Modul vorgesehen sein. Dies ist wie auch der Betrachtungsabschnitt-Sensor 112a an einer Halterung befestigt, an der sich die Düse befindet, welche von der dem betreffenden Unterabschnitt des Ausgangs angesteuert wird. In diesem Fall sind die Unterabschnitte des Ausgangs 160, der Abbildungseinheit 150 und des Vergleichers für jeden Betrachtungsabschnitt-Sensor 112a autonom. Der Betrachtungsabschnitt-Sensor 112a und die zugehörigen Unterabschnitte können in dem gleichen Gehäuse montiert sein.

Die Figur 2 ist lediglich eine schematische Funktionsdarstellung und ist nicht geeignet, körperliche Eigenschaften und Anordnungen zu definieren.

## Patentansprüche

1. Verfahren zur Ermittlung eines Unkrautanteils in einem Betrachtungsabschnitt (10) eines Feldes (20) mit den Schritten:
- Erfassen einer Ist-Spektralinformation mit mindestens einem optischen Betrachtungsabschnitt-Sensor (12a, b), der auf den Betrachtungsabschnitt (10) gerichtet ist;
- Erfassen einer Referenz-Spektralinformation mittels eines optischen Referenzsensors (32), der auf einen Referenzabschnitt (30) der gleichen Bodenfläche (22) gerichtet ist;
- Ermitteln eines Unterschieds zwischen der Ist-Spektralinformation und der Referenz-Spektralinformation, und
- Abbilden des Unterschieds auf den Unkrautanteil mittels einer vorgegebenen Abbildung.

2. Verfahren nach Anspruch 1, wobei der Referenzabschnitt (30) und der Betrachtungsabschnitt (10) Bereiche desselben landwirtschaftlichen Feldes (20) sind, nahe beieinander angeordnet sind, oder sich zumindest teilweise überlappen.

3. Verfahren nach Anspruch 1 oder 2, wobei der Referenzsensor (32') an einer Fläche des Feldes (20) befestigt ist, über die der Betrachtungsabschnitt (10) bzw. der mindestens eine optische Betrachtungsabschnitt-Sensor (12a, b) geführt wird,

4. Verfahren nach einem der vorangehenden Ansprüche, wobei die Ist-Spektralinformation und die Referenz-Spektralinformation zumindest ein Spektrumsintervall charakterisieren, insbesondere ein Spektrumsintervall, welches einen sichtbaren Bereich und/oder einen Infrarot- und/oder UV-Bereich umfasst, wobei die Ist-Spektralinformation und die Referenz-Spektralinformation jeweils als ein Wert oder als Folge von Werten, die unterschiedliche Wellenlängen betreffen, vorliegt.

5. Verfahren nach einem der vorangehenden Ansprüche, wobei die Ist-Spektralinformation und/oder die Referenz-Spektralinformation erfasst werden mittels Farbsensoren (12a, b; 32) oder mittels elektronischer Kameras, wobei den Kameras eine Farberfassungseinheit nachgeschaltet ist, die Farbwerte über das gesamte Bild oder über einen Bildabschnitt der Kameras ermittelt.

6. Verfahren nach einem der vorangehenden Ansprüche, wobei ferner Abschattungen des Betrachtungsabschnitts und des Referenzabschnitts ermittelt werden und bei unterschiedlichen Abschattungen die Ist- oder die Referenz-Spektralinformation oder der Unterschied zwischen der Ist- und der Referenz-Spektralinformationen durch arithmetische Kombination mit einer vorgegebenen Beleuchtungskorrekturvariablen angepasst werden.

7. Verfahren nach einem der vorangehenden Ansprüche, wobei das Verfahren ferner den folgenden Schritt aufweist:
- Ausrichten des Referenzsensors (32; 32') auf ein Referenzgebiet, welches keinen Unkrautanteil oder einen tolerierbaren Unkrautanteil aufweist.

8. Landtechnik-Steuereinrichtung (100) mit
- mindestens einem optischen Betrachtungsabschnitt-Sensor (112a, b)
- einer Referenzsensor-Empfangsschnittstelle (133) und einem optischen Referenzsensor (132), wobei der Betrachtungsabschnitt-Sensor (112a, b) und die Referenzsensor-Empfangsschnittstelle (133) an dem gleichen Fahrzeug befestigt sind;
- einem Vergleicher (140), der den Sensoren (112a, b; 132, 132') nachgeschaltet ist und eingerichtet ist, Ist-Spektralinformation des mindestens einen Betrachtungsabschnitt-Sensors (112a, b) mit Referenz-Spektralinformation des optischen Referenzsensors (132, 132') zu vergleichen; und
- einer Abbildungseinheit (150), die dem Vergleicher (140) nachgeschaltet ist und eingerichtet ist, das Vergleichsergebnis des Vergleichers (160) auf einen Unkrautanteil abzubilden und an einem Ausgang (160) der Landtechnik-Steuereinrichtung (100) abzugeben.

9. Landtechnik-Steuereinrichtung (100) nach Anspruch 8, wobei die Referenzsensor-Empfangsschnittstelle (133) eingerichtet ist, mit dem als statischen Referenzsensor (132') ausgebildeten optischen Referenzsensor über eine drahtlose Datenverbindung verbunden zu sein.

10. Landtechnik-Steuereinrichtung nach Anspruch 8 oder 9, wobei dem mindestens einen optischen Betrachtungsabschnitt-Sensor (112a, b) und/oder der Referenzsensor-Empfangsschnittstelle (133) eine Farberfassungseinheit (170) nachgeschaltet sind, wobei der Referenzsensor (132) bzw. der mindestens eine optische Betrachtungsabschnitt-Sensor (112 a, b) zur Aufnahme bzw. die Empfangsschnittstelle (133) zum Empfang eines elektronischen Bildes ausgebildet sind.

## Claims

1. Method for determining a weed percentage in an observation section (10) of a field (20), having the steps of:
- acquiring an item of actual spectral information using at least one optical observation section sensor (12a, b) which is aimed at the observation section (10);
- acquiring an item of reference spectral information using an optical reference sensor (32) which is aimed at a reference section (30) of the same ground area (22);
- determining a difference between the actual spectral information and the reference spectral information, and
- mapping the difference to the weed percentage using predefined mapping.

2. Method according to Claim 1, the reference section (30) and the observation section (10) being regions of the same agricultural field (20), being arranged close together or at least partially overlapping.

3. Method according to Claim 1 or 2, the reference sensor (32') being fastened on an area of the field (20) over which the observation section (10) or the at least one optical observation section sensor (12a, b) is guided.

4. Method according to one of the preceding claims, the actual spectral information and the reference spectral information characterizing at least one spectrum interval, in particular a spectrum interval which comprises a visible region and/or an infrared and/or UV region, the actual spectral information and the reference spectral information each
being present as a value or as a sequence of values relating to different wavelengths.

5. Method according to one of the preceding claims, the actual spectral information and/or the reference spectral information being acquired using color sensors (12a, b; 32) or electronic cameras, a color detection unit which determines color values over the entire image or over an image section from the cameras being connected downstream of the cameras.

6. Method according to one of the preceding claims, shading of the observation section and of the reference section also being determined, and, in the case of different shading, the actual or reference spectral information or the difference between the actual and reference spectral information being adapted by means of arithmetic combination with a predefined lighting correlation variable.

7. Method according to one of the preceding claims, the method also having the following step of:
- aligning the reference sensor (32; 32') with a reference area which does not have a weed percentage or has a tolerable weed percentage.

8. Agricultural control device (100) having
- at least one optical observation section sensor (112a, b),
- a reference sensor receiving interface (133) and an optical reference sensor (132), the observation section sensor (112a, b) and the reference sensor receiving interface (133) being fastened to the same vehicle;
- a comparator (140) which is connected downstream of the sensors (112a, b; 132, 132') and is set up to compare actual spectral information from the at least one observation section sensor (112a, b) with reference spectral information from the optical reference sensor (132, 132'); and
- a mapping unit (150) which is connected downstream of the comparator (140) and is set up to map the comparison result from the comparator (160) to a weed percentage and to output it at an output (160) of the agricultural control device (100).

9. Agricultural control device (100) according to Claim 8, the reference sensor receiving interface (133) being set up to be connected to the optical reference sensor in the form of a static reference sensor (132') via a wireless data connection.

10. Agricultural control device according to Claim 8 or 9, a color detection unit (170) being connected downstream of the at least one optical observation section sensor (112a, b) and/or the reference sensor receiving interface (133), the reference sensor (132) or the at least one optical observation section sensor (112a, b) being designed to record an electronic image and the receiving interface (133) being designed to receive an electronic image.

## Revendications

1. Procédé pour la détermination de la quantité de plantes adventices dans une partie observée (10) d'un champ (20), comprenant les étapes suivantes:
- détecter une information spectrale réelle avec au moins un capteur optique de partie observée (12a, b), qui est dirigé vers la partie observée (10);
- détecter une information spectrale de référence au moyen d'un capteur de référence optique (32), qui est dirigé vers une partie de référence (30) de la même parcelle de terrain (22);
- déterminer une différence entre l'information spectrale réelle et l'information spectrale de référence, et
- représenter la différence sur la quantité de plantes adventices au moyen d'une représentation prédéterminée.

2. Procédé selon la revendication 1, dans lequel la partie de référence (30) et la partie observée (10) sont des zones du même champ agricole (20), sont disposées à proximité l'une de l'autre, ou se recouvrent au moins partiellement.

3. Procédé selon la revendication 1 ou 2, dans lequel le capteur de référence (32') est fixé à une surface du champ (20), sur laquelle la partie observée (10) ou ledit au moins un capteur optique de partie observée (12a, b) est conduit.

4. Procédé selon l'une quelconque des revendications précédentes, dans lequel l'information spectrale réelle et l'information spectrale de référence caractérisent au moins un intervalle de spectre, en particulier un intervalle de spectre, qui comprend un domaine visible et/ou un domaine infrarouge et/ou UV, dans lequel l'information spectrale réelle et l'information spectrale de référence se présentent respectivement comme une valeur ou comme une suite de valeurs, qui concernent différentes longueurs d'onde.

5. Procédé selon l'une quelconque des revendications précédentes, dans lequel on détecte l'information spectrale réelle et/ou l'information spectrale de référence au moyen de détecteurs de couleurs (12a, b; 32) ou au moyen de caméras électroniques, dans lequel une unité de détection des couleurs, qui détermine les valeurs de couleurs sur toute l'image ou sur une partie d'image, est montée après les caméras.

6. Procédé selon l'une quelconque des revendications précédentes, dans lequel on détecte en outre des ombres de la partie observée et de la partie de référence et en cas d'ombres différentes on adapte l'information spectrale réelle ou l'information spectrale de référence ou la différence entre l'information spectrale réelle et l'information spectrale de référence par combinaison arithmétique avec une variable de correction d'éclairement prédéterminée.

7. Procédé selon l'une quelconque des revendications précédentes, dans lequel le procédé présente en outre l'étape suivante:
- diriger le capteur de référence (32; 32') vers une zone de référence, qui ne présente pas de plantes adventices ou présente une quantité tolérable de plantes adventices.

8. Dispositif de commande agricole (100) avec
- au moins un capteur optique de partie observée (112a, b),
- une interface de réception de capteur de référence (133) et un capteur de référence optique (132), dans lequel le capteur de partie observée (112a, b) et l'interface de réception de capteur de référence (133) sont fixés sur le même véhicule;
- un comparateur (140), qui est monté après les capteurs (112a, b; 132, 132') et qui est conçu pour comparer l'information spectrale réelle dudit au moins un capteur de partie observée (112a, b) avec l'information spectrale de référence du capteur de référence optique (132, 132'); et
- une unité de représentation (150), qui est montée après le comparateur (140) et qui est conçue pour représenter le résultat de la comparaison du comparateur (160) sur une quantité de plantes adventices et pour le produire à une sortie (160) du dispositif de commande agricole (100).

9. Dispositif de commande agricole (100) selon la revendication 8, dans lequel l'interface de réception de capteur de référence (133) est conçue pour être reliée au capteur de référence optique formé par un capteur de référence statique (132') par l'intermédiaire d'une liaison de données sans fil.

10. Dispositif de commande agricole selon la revendication 8 ou 9, dans lequel une unité de détection des couleurs (170) est montée après ledit au moins un capteur optique de partie observée (112a, b) et/ou l'interface de réception (133) de capteur de référence (170), dans lequel le capteur de référence (132) ou ledit au moins un capteur optique de partie observée (112a, b) est configuré pour l'enregistrement ou l'interface de réception (133) est configurée pour la réception d'une image électronique.
